# EUROPEAN PATENT APPLICATION

(11) **EP 2 309 002 A1**
(43) Date of publication of application: **13.04.2011**
(21) Application number: 09305913.7
(22) Date of filing: 29.09.2009
(51) Int. Cl.: C12Q 1/68

(54) **Signature for the diagnosis of colorectal cancer aggressiveness**

(71) Applicant: Centre National de la Recherche Scientifique (CNRS), 75016 Paris (FR)
(72) Inventor: The designation of the inventor has not yet been filed
(74) Representative: Warcoin, Jacques

(57) **Abstract**

The present invention relates to a method for diagnosing aggressiveness of a colorectal cancer in a patient from a colorectal cancer sample of said patient, comprising: a) measuring in vitro the expression level of genes in said patient colorectal cancer sample and in a healthy colorectal tissue sample, wherein said genes are: POLQ, CDC45, CDC6, CDT1, SLD5, MCM2 and MCM7; b) calculating for each gene an expression level ratio of the expression level in said patient colorectal cancer sample to the expression level in said healthy colorectal tissue sample; c) comparing each gene expression level ratio to a corresponding threshold value, and d) diagnosing colorectal cancer aggressiveness if the ratios of POLQ and at least 3 other genes are superior to their corresponding threshold values. Dedicated microarrays and kits are also described, as well as a method of selecting a suitable treatment.

## Description

### FIELD OF THE INVENTION

The present invention is in the field of colorectal cancer management, including diagnosis of aggressiveness and genetic instability, and selection of an appropriate treatment. The invention is based on the finding that over-expression of POLQ and of at least 3 genes selected from CDC45, CDC6, CDT1, SLD5, MCM2 and MCM7 is highly correlated to aggressiveness and thus to survival.

### BACKGROUND ART

With 655,000 deaths worldwide per year, colorectal cancer is the third most common form of cancer and the second leading cause of cancer-related death in the Western world. Management of colorectal cancer patients and selection of the best treatment protocol depending on tumor aggressiveness is thus a crucial health problem.

The main options for colorectal cancer therapy are surgery, with or without adjuvant chemotherapy and/or radiotherapy, depending on the individual patient's staging and other medical factors.

The selection of an appropriate treatment is crucial for the patient and also for economical reasons. Indeed, for patient survival, it is essential to know when to use immediately a heavy and aggressive treatment protocol in order to prevent extension of an aggressive colorectal cancer. Otherwise, the survival of the patient may be highly compromised. In contrast, performing a heavy and aggressive treatment when it is not necessitated by the tumor carried by the patient is also disadvantageous for the patient. Indeed, heavy and aggressive treatments always lead to adverse toxicities that may significantly affect the patient's quality of life. In addition, since colorectal cancer mainly affects the elderly, such toxicities may even in some circumstances threaten the patient's life. Moreover, such heavy and aggressive treatments are usually very costly, and should thus be performed only when it is necessary.

There is thus a great need for tests permitting to assess the aggressiveness of a particular colorectal tumor, in order to be able to select the best treatment.

Currently, treatment selection is based on tumor staging, which is usually performed using the Tumor/Node/Metastasis (TNM) test from the American Joint Committee on Cancer (AJCC). The TNM system assigns a number based on three categories. "T" denotes the degree of invasion of the intestinal wall, "N" the degree of lymphatic node involvement, and "M" the degree of metastasis. The broader stage of a cancer is usually quoted as a number I, II, III, IV derived from the TNM value grouped by prognosis; a higher number indicates a more advanced cancer and likely a worse outcome. Details of this system are in Table 1 below:

**Table 1. TNM test staging**

| **AJCC stage** | **TNM stage** | **TNM stage criteria for colorectal cancer** |
|---|---|---|
| Stage 0 | Tis N0 M0 | Tis: Tumor confined to mucosa; cancer-*in-situ* |
| Stage I | T1 N0 M0 | T1: Tumor invades submucosa |
| Stage I | T2 N0 M0 | T2: Tumor invades muscularis propria |
| Stage II-A | T3 N0 M0 | T3: Tumor invades subserosa or beyond (without other organs involved) |
| Stage II-B | T4 N0 M0 | T4: Tumor invades adjacent organs or perforates the visceral peritoneum |
| Stage III-A | T1-2 N1 M0 | N1: Metastasis to 1 to 3 regional lymph nodes. T1 or T2. |
| Stage III-B | T3-4 N1 M0 | N1: Metastasis to 1 to 3 regional lymph nodes. T3 or T4. |
| Stage III-C | any T, N2 M0 | N2: Metastasis to 4 or more regional lymph nodes. Any T. |
| Stage IV | any T, any N, M1 | M1: Distant metastases present. Any T, any N. |

While this test and staging system provides some valuable information concerning the stage at which colorectal cancer has been diagnosed in the patient, it does not give information on the tumor aggressiveness and its usefulness for prognosis is thus limited. Indeed, while it is clear that patients at stage IV have a bad prognosis, the fact that colorectal cancer is diagnosed at an early stage does not preclude the possibility that the tumor, although at its earliest stage, may further develop very rapidly. In particular, it is totally unknown why 20 to 40% of patients with a stage II colorectal cancer (i.e., early cancer without metastasis nor lymph node invasion at diagnosis) will rapidly worsen and die. In contrast, when lymph nodes are invaded by tumor cells, the TNM test gives a bad prognosis and the patient is usually subjected to heavy surgery followed by intensive chemotherapy. However, if some of these patients were actually suffering from a non aggressive colorectal cancer, chemotherapy, which is an aggressive and mutagenic treatment, might be avoided without decreasing the survival prognosis of the patient.

There is thus a real need for better prognosis tests of colorectal cancer, not only to improve patient global survival, but also to improve their quality of life and to keep aggressive and costly chemotherapies for patients who will really benefit from them. The finding of new and more reliable prognosis tests is thus a very active research field.

In normal cell proliferation, DNA replication is performed by 3 DNA polymerases known as replicating polymerases (POLA, POLD and POLE). However, 10 other DNA polymerases have been identified in human cells, which are known as specialized polymerases and which functions are still largely unknown. These specialized polymerases appear to have been maintained through evolution because of their ability to process despite of DNA damages. It also appears that these polymerases are very mutagenic and that their activity is tightly controlled.

POLQ (also known as POL theta or POL θ) is one of these specialized DNA polymerases, and contains a helicase domain in its N-terminal portion and a polymerase domain in its C-terminus. Although the function of this particular specialized DNA polymerase is still largely unknown, it appears to be involved in maintenance of genome stability and in DNA repair.

Kawamura et al (Kawamura et al., 2004) have compared the expression level of POLQ in non-tumor tissue and in lung (27 patients), colon (26 patients) and stomach (28 patients) cancer tissue. They found that POLQ is only barely detectable in non-tumor tissue, but that it is over-expressed in a significant proportion of lung, stomach and cancer tissues. The authors also indicate that over-expression of POLQ in tumor tissue is correlated to poor survival prognosis.

The inventors analyzed the variation of expression of 47 DNA replication genes in tumor versus adjacent normal colorectal tissues and compared these data with disease progression and clinical features. They confirmed that POL Q was significantly over-expressed in a significant proportion of tumor tissues, mainly at early stages. However, contrary to Kawamura et al, they found no significant correlation between POLQ overexpression alone and survival prognosis. This discrepancy is probably linked to the fact that the number of colorectal cancer patients analyzed in Kawamura (26 patients) was not sufficient (the inventors analyzed 74 patients) and to the absence of true statistical analysis in Kawamura et al.

However, the inventors found that, although POLQ alone may not be used as a prognosis marker of colorectal cancer, another test necessitating the analysis of the expression of POLQ and several other genes involved in the firing of DNA replication, permits to obtain a significant correlation with survival prognosis and may thus be used as a prognosis test. Indeed, the inventors found that over-expression of POLQ, if and only if associated with over-expression of at least 3 genes selected from CDC45, CDC6, CDT1, SLD5, MCM2 and MCM7, was significantly associated with tumor aggressiveness and thus with bad prognosis.

### DESCRIPTION OF THE INVENTION

The present invention thus relates to a method for diagnosing aggressiveness of a colorectal cancer in a patient from a colorectal cancer sample of said patient, comprising:
a) measuring in vitro the expression level of genes in said patient colorectal cancer sample and in a healthy colorectal tissue sample, wherein said genes are: POLQ, CDC45, CDC6, CUT1, SLD5, MCM2 and MCM7.
b) calculating for each gene an expression level ratio of the expression level in said patient colorectal cancer sample to the expression level in said healthy colorectal tissue sample,
c) comparing each gene expression level ratio to a corresponding threshold value, and
d) diagnosing colorectal cancer aggressiveness if the ratios of POLQ and at least 3 other genes are superior to their corresponding threshold values.

Data concerning the above mentioned human genes are provided in following Table 2:

**Table 2**

| Symbol | Full name | Other aliases | Entrez GenelD | mRNA reference sequence | Protein reference sequence |
|---|---|---|---|---|---|
| POLQ | polymerase (DNA directed), theta | DKFZp781 A0112 , POLH, PR00327 | 10721 | NM_199420.3 | NP_955452.3 |
| CDC45L (or CDC45) | CDC45 cell division cycle 45-like | CDC45, CDC45L2, PORC-PI-1 | 8318 | NM_003504.3 | NP_003495.1 |
| CDC6 | cell division cycle 6 homolog | | 990 | NM_001254.3 | NP_001245.1 |
| CDT1 | chromatin licensing and DNA replication factor 1 | DUP, RIS2 | 81620 | NM_030928.3 | NP_112190.2 |
| GINS4 (or SLDS) | GINS complex subunit 4 (Sld5 homolog) | MGC 14799, SLD5 | 84296 | NM_032336.2 | NP_115712.1 |
| MCM2 | minichromosome maintenance complex component 2 | BM28; CCNL1; CDCL1; cdc19; D3S3194; MITOTIN; KIAA0030; MGC10606 | 4171 | NM_004526.2 | NP_004517.2 |
| MCM7 | minichromosome maintenance complex component 7 | CDC47; P85MCM; P1CDC47; PNAS-146; CDABP0042; P1.1-MCM3 | 4176 | NM_005916.3 NM_182776.1 | NP_005907.3 NP_877577.1 |

According to the present invention, "aggressiveness" of a colorectal cancer is intended to mean the propensity of said colorectal cancer to invade the intestinal wall, lymph nodes and to generate metastases and the rapidity with which said invasions may appear. Aggressiveness of the colorectal cancer is obviously correlated to survival, and the above method may be used for prognosing survival of the patient, in which case diagnosing of aggressiveness results in a bad survival prognosis and diagnosis of the absence of aggressiveness results in a good survival prognosis.

The above methods are performed using a colorectal cancer sample of the patient to be tested. In some cases, the methods according to the invention may further comprise a preliminary step of taking a colorectal cancer sample from the patient. By a "colorectal cancer sample", it is referred to a tumor colorectal tissue sample. Indeed, even in a cancerous patient, colorectal tissue still comprises non tumor healthy tissue. The "colorectal cancer sample" should thus be limited to tumor colorectal tissue taken from the patient. Said "colorectal cancer sample" may be a biopsy sample or a sample taken from a surgical colorectal resection therapy.

In addition, the methods according to the invention may comprise another preliminary step, between the taking of the sample from the patient and steps a) as defined above, corresponding to the transformation of the colorectal cancer sample (and optionally of the healthy colorectal tissue sample) into a mRNA (or corresponding cDNA) sample or into a protein sample, which is then ready to use for in vitro measuring of genes expression levels in steps a). Preparation or extraction of mRNA (as well as retrotranscription into cDNA) or proteins from a tissue sample is only routine procedure well known to those skilled in the art.

Once a ready to use colorectal cancer mRNA (or corresponding CDNA) or protein sample is available, the measure of POLQ, CDC45, CDC6, CDT1, SLD5, MCM2 and MCM7 genes expression levels may be performed, depending on the type of transformation and the available ready to use sample, either at the mRNA (i.e. based on the mRNA content of the sample) or at the proteic level (i.e. based on the protein content of the sample). In some embodiments, the expression levels of some of the genes may be measured at the mRNA level, while the expression levels of other genes are measured at the proteic level. In this case, part of the colorectal cancer sample taken from the patient has been transformed into a mRNA (or corresponding cDNA) sample and another part has been transformed into a protein sampleIn other embodiments, the expression levels of all tested genes are measured either at the mRNA or at the proteic level.

When expression levels are measured at the mRNA level, it may be notably performed using well known technologies such as quantitative PCR or nucleic acid microarray technologies (including cDNA and oligonucleotide microarrays). These technologies are now used routinely by those skilled in the art and thus do not need to be detailed here. Examples of embodiments using quantitative PCR are described in the experimental section. Alternatively, any known or future technology permitting to assess genes expression levels based on mRNA contents may be used. For instance, tissue microarrays coupled to fluorescent in situ hybridization may be used. Tissue microarrays (also TMAs) consist of paraffin blocks in which up to 1000 separate tissue cores are assembled in array fashion to allow multiplex histological analysis. In the tissue microarray technique, a hollow needle is used to remove tissue cores as small as 0.6 mm in diameter from regions of interest in paraffin-embedded tissues such as clinical biopsies or tumor samples. These tissue cores are then inserted in a recipient paraffin block in a precisely spaced, array pattern. Sections from this block are cut using a microtome, mounted on a microscope slide and then analyzed by any method of standard histological analysis. Each microarray block can be cut into 100 - 500 sections, which can be subjected to independent tests. Tests commonly employed in tissue microarray include immunohistochemistry, and fluorescent in situ hybridization. For analysis at the mRNA level, tissue microarray technology may be coupled to fluorescent in situ hybridization.

When expression levels are measured at the proteic level, it may be notably performed using specific antibodies, in particular using well known technologies such as western blot, ELISA or ELISPOT, antibodies microarrays, or tissue microarrays coupled to immunohistochemistry.

The genes expression levels are also measured in a healthy colorectal tissue sample, which is used as a healthy control. Indeed, as mentioned above, the inventors found that POLQ is barely detectable in healthy colorectal tissue, and over-expressed in a proportion of colorectal cancer tissues, in which case, if and only if associated to overexpression of at least 3 other DNA repair firing genes also over-expressed in a proportion of colorectal cancer tissues, it is significantly correlated to tumor aggressiveness and bad prognosis. As a result, the low or high expression of POLQ and other genes in a patient colorectal cancer sample is determined by comparison to the expression in a control healthy colorectal tissue sample. Said healthy colorectal tissue sample may be composed of only one subject's healthy colorectal tissue or may be a pooled sample made of several subjects' healthy colorectal tissues. When the sample is made of only one subject's healthy colorectal tissue, said subject may be either the tested patient or another subject. Indeed, as mentioned above, even in a cancerous patient, colorectal tissue still comprises non tumor healthy tissue. In particular, when the colorectal cancer sample is taken from a surgical resection therapy, adjacent non tumor healthy tissue of the patient to be diagnosed is generally available and may be used as healthy control sample. However, as indicated above, a healthy control sample made of healthy colorectal tissue from one or more other subjects may be used instead. Said healthy colorectal tissue sample may also be preliminary transformed into a mRNA (or corresponding cDNA) or protein sample.

The comparison of the expression levels of the measured genes in said patient's colorectal cancer sample and in the control healthy colorectal tissue sample is made by calculating an expression level ratio of the expression level in said patient's colorectal cancer sample to the expression level in the control healthy colorectal tissue sample, and by comparing the obtained expression level ratio to a corresponding threshold value. According to the present invention, a "threshold value" is intended to mean a value that permits to discriminate samples in which the expression level ratio of the gene of interest corresponds to an expression level of said gene of interest in the patient's colorectal cancer sample that is low or high. In particular, if a gene expression level ratio is inferior or equal to the threshold value, then the expression level of this gene in the patient's colorectal cancer sample is considered low, whereas if a gene expression level ratio is superior to the threshold value, then the expression level of this gene in the patient's colorectal cancer sample is considered high. For each gene, and depending on the method used for measuring the expression level of the genes, the optimal threshold value may vary. However, it may be easily determined by a skilled artisan based on the analysis of a control healthy colorectal tissue sample and several control colorectal cancer samples in which the expression level (low or high) is known for this particular gene. In some cases, a unique threshold value, which is relevant for all tested genes, may be used and thus all expression level ratios are then compared to the same threshold value. In particular, the inventors determined that a unique threshold value of 5 is particularly useful, notably when the expression level of all genes is measured at the mRNA level using quantitative PCR.

The present invention further relates to a microarray dedicated to the implementation of the methods according to the invention, comprising at most 500, preferably at most 300, at most 200, more preferably at most 150, at most 100, even more preferably at most 75, at most 50, at most 40, at most 30, at most 20, at most 10 distinct probes, at least 7 of which specifically bind to POLQ, CDC45, CDC6, CUT1, SLD5, MCM2 and MCM7 mRNAs (or corresponding cDNAs) or proteins.

In a preferred embodiment, said microarray is a nucleic acid microarray, comprising at most 500, preferably at most 300, at most 200, more preferably at most 150, at most 100, even more preferably at most 75, at most 50, at most 40, at most 30, at most 20, at most 10 distinct probes (thus excluding for instance pangenomic microarrays), at least 7 of which specifically hybridize to POLQ, CDC45, CDC6, CDT1, SLD5, MCM2 and MCM7 mRNAs (or corresponding cDNAs). According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray", the oligonucleotides being about 25 to about 60 base pairs or less in length).

Alternatively, in another embodiment, said microarray may be an antibodies microarray, comprising at most 500, preferably at most 300, at most 200, more preferably at most 150, at most 100, even more preferably at most 75, at most 50, at most 40, at most 30, at most 20, at most 10 distinct antibodies, at least 7 of which specifically bind to POLQ, CDC45, CDC6, CDT1, SLD5, MCM2 or MCM7 proteins.

Alternatively to nucleic acid or antibody microarray technology, quantitative PCR may be used and amplification primers specific for the genes to be tested are thus also very useful for performing the methods according to the invention. The present invention thus further relates to a kit for diagnosing aggressiveness of a colorectal cancer in a patient from a colorectal cancer sample of said patient, comprising a dedicated microarray as described above or amplification primers specific for POLQ, CDC45, CDC6, CDT1, SLD5, MCM2 and MCM7. Here also, when the kit comprises amplification primers, while said kit may comprise amplification primers specific for other genes, said kit preferably comprises at most 100, at most 75, 50, at most 40, at most 30, preferably at most 25, at most 20, at most 15, more preferably at most 10, at most 8, at most 6, even more preferably at most 5, at most 4, at most 3 or even 2 or one or even zero couples of amplification primers specific for other genes than POLQ, CDC45, CDC6, CUT1, SLD5, MCM2 and MCM7. Such a kit may further comprise a healthy colorectal tissue mRNA or cDNA sample. In some embodiments, said kit may also further comprise one or more control samples corresponding to colorectal cancer samples for which the low or high expression level of POLQ, CDC45, CDC6, CUT1, SLD5, MCM2 and MCM7 is known. Such control samples may be used for determining an optimal threshold value for each tested gene or all tested genes.

As mentioned in the introduction, the capacity of prognosing colorectal cancer evolution, which is linked to its aggressiveness, is very important for selecting a suitable treatment, since heavy and costly treatments with potentially severe adverse effects should be used, in addition to traditional surgical treatment, each time they are necessary, but only when they are necessary. The present invention thus also concerns a method for choosing a suitable treatment of colorectal cancer in a patient, comprising:
a) diagnosing or not aggressiveness of said colorectal cancer in said patient using the method according to the invention as described above, and
b) adding adjuvant radiotherapy or chemotherapy to surgical treatment if said colorectal cancer is diagnosed as aggressive in step a).

### DESCRIPTION OF THE FIGURES

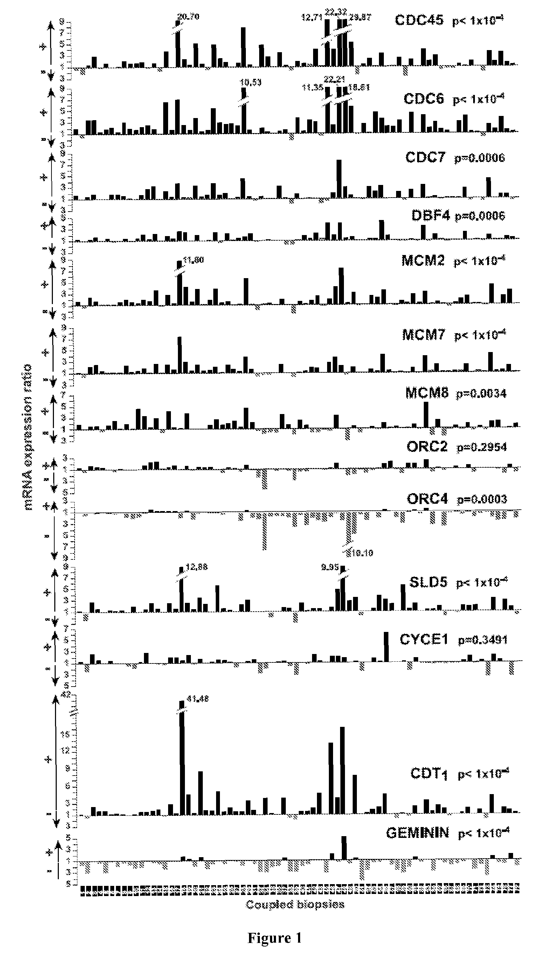

**Figure 1****. mRNA expression ratios (T/N) of initiation genes in the 74 tumors and adjacent control tissues.** T (tumor)/ N(normal) mRNA expression ratios between normalized values were calculated showing that firing/licensing genes are up-regulated in colon cancers. T/N>1 indicates higher expression in the tumor than in the adjacent normal tissue. T/N <1 means a lower expression in cancers than in control tissues. For graph representation T/N values lower than 1 were transformed into the inverse N/T values. The sample number is indicated in the black boxes underneath the x axis. The p-values from the bilateral exact binomial test (change from normal) are given uncorrected; the significance level is evaluated using the Benjamini Krieger 2001 procedure for an overall FDR of 0.05.

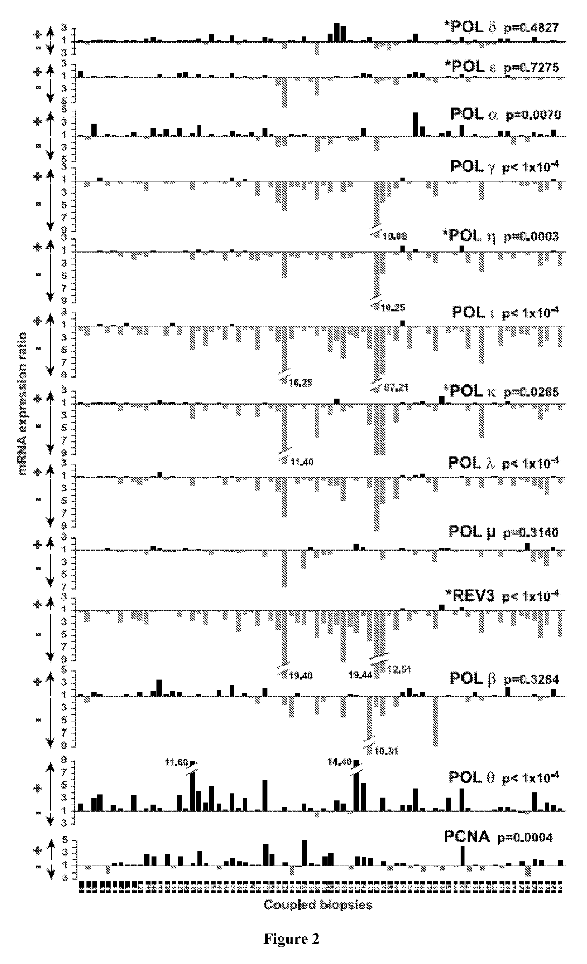

**Figure 2****. mRNA expression ratios (T/N) of DNA polymerases in the 74 tumours and adjacent control tissues.** T/N mRNA expression ratios between normalized values were calculated. T/N>1 indicates a higher expression in the tumour sample compared to the adjacent normal tissue. T/N <1 means a lower expression in cancers than in control tissues. For graph representation T/N values lower than 1 were transformed into the inverse N/T values. The sample number is indicated in the black boxes underneath the x axis. The p-values from the bilateral exact binomial test (change from normal) are given uncorrected; the significance level is evaluated using the Benjamini Krieger 2001 procedure for an overall FDR of 0.05.

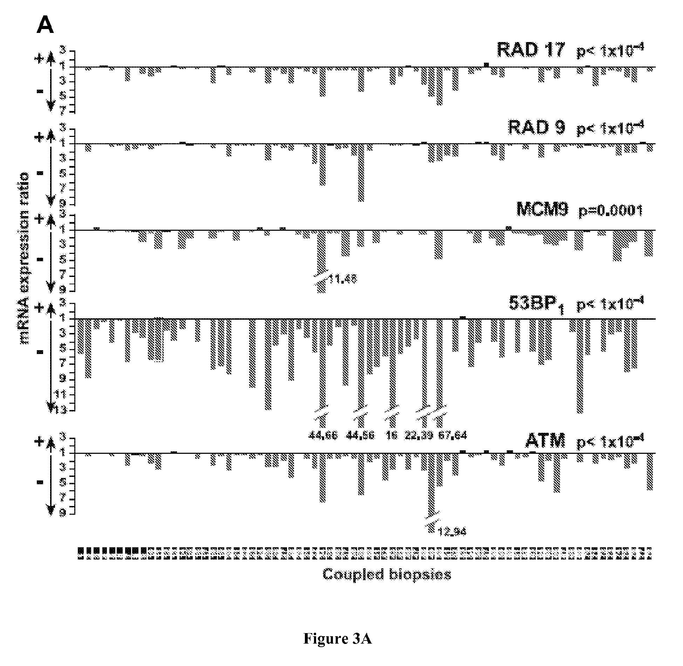

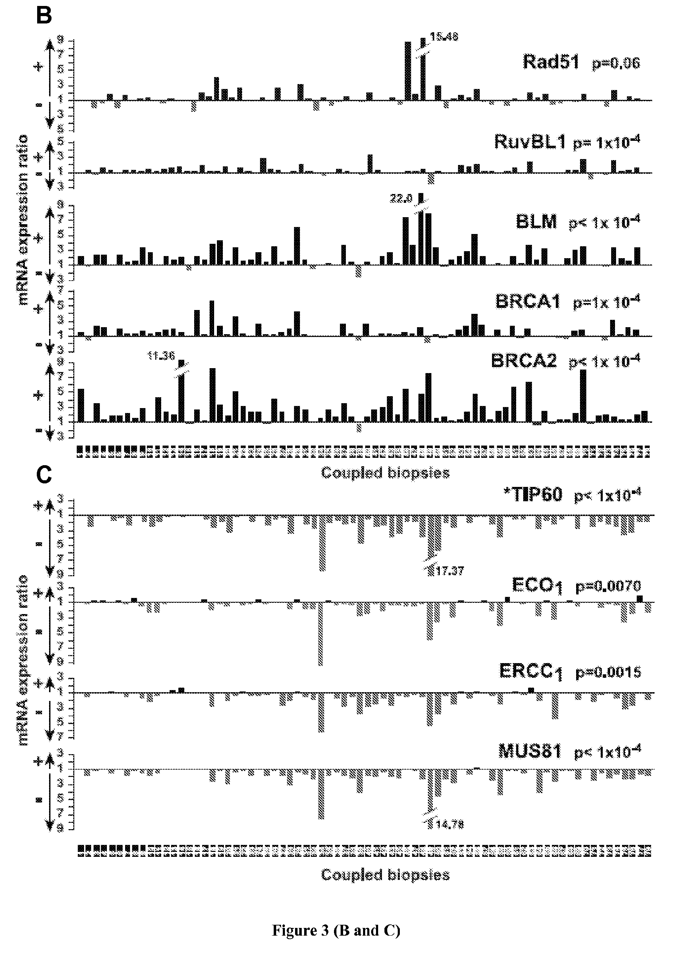

**Figure 3****. mRNA expression ratios (T/N) of genes involved in S-phase checkpoint signaling, protection of DNA replication forks and other DNA transactions in the 74 tumours and adjacent control tissues.** T/N mRNA expression ratios between normalized values were calculated. T/N>1 indicates a higher expression in the tumour sample compared to the adjacent normal tissue. T/N <1 means a lower expression in cancers than in control tissues. For graph representation T/N values lower than 1 were transformed into the inverse N/T values. The sample number is indicated in the black boxes underneath the x axis. The p-values from the bilateral exact binomial test (change from normal) are given uncorrected; the significance level is evaluated using the Benjamini Krieger 2001 procedure for an overall FDR of 0.05. (A) Genes involved in S-phase checkpoint signaling and (B) in the maintenance of DNA replication forks; (C) DNA repair and cohesion genes.

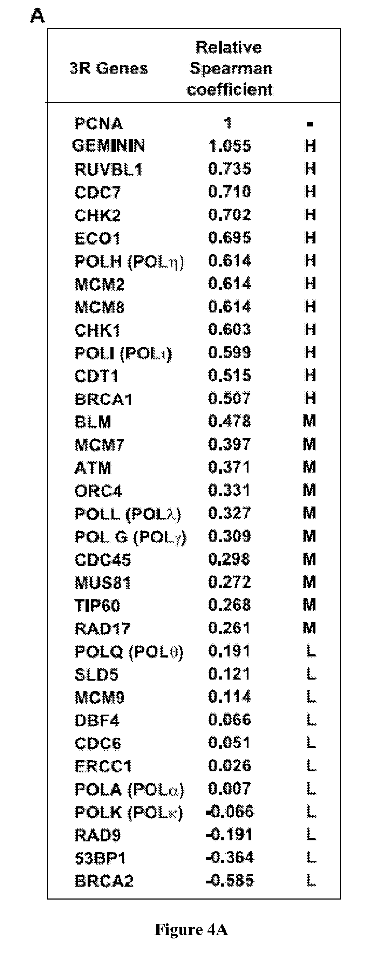

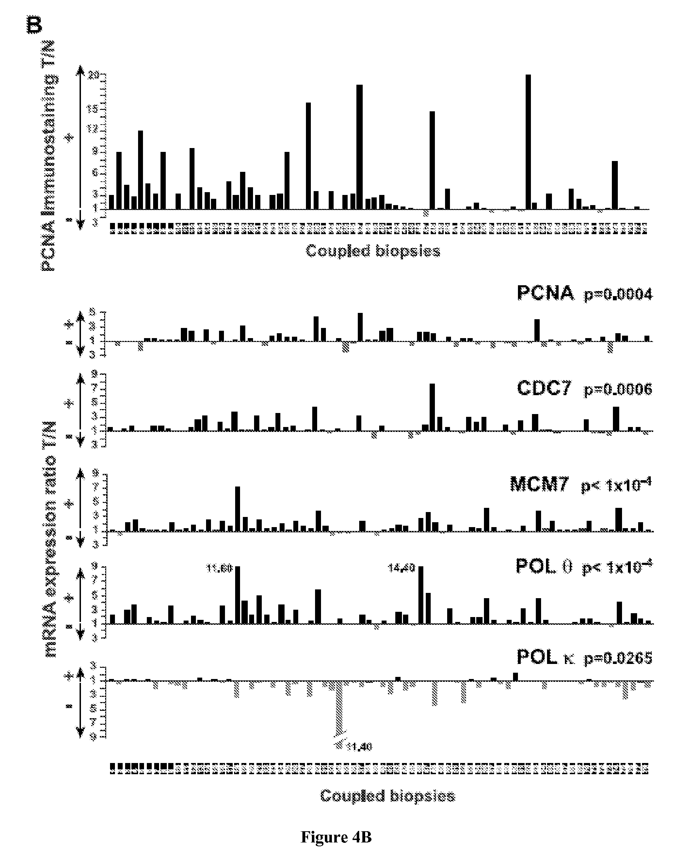

**Figure 4****. Correlation between the expression in tumors of representative replication genes expression (T/N ratios) and parallel PCNA immunostaining on TMA, assessed by using a non parametric Spearman correlation test.** (A) Relative spearman coefficients were calculated by normalizing to PCNA. H, M and L mean high (rho>0.5), moderate (0.5<rho<0.25) and low (rho<0.25) correlation between gene deregulation and proliferation status (i.e. percentage of PCN-immunostained cells in tumors), respectively. The numbers of expression ratios analysed for each gene are indicated by n in Table 1. (B) Protein or mRNA expression T/N ratios of PCNA and representative replication genes displaying igh (*CDC7*), moderate (*MCM7*) and low (*POLQ* and *POLK*) correlation with the PCNA staining.

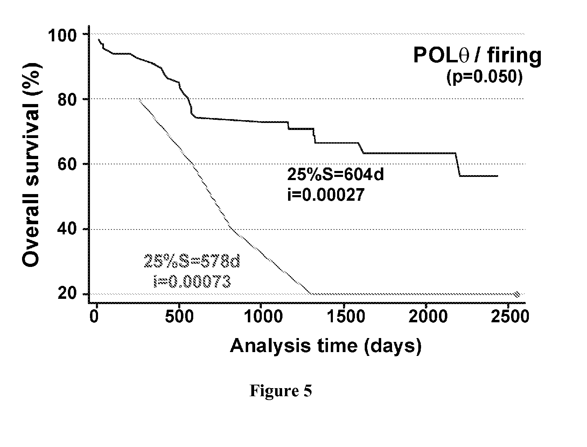

**Figure 5****. Incidence of the co-expression of POLQ and at least three licensing/initiating replication genes on overall survival.** Kaplan-Meier's analysis of the overall survival among the 74 patients according to whether POL Q and three DNA replication genes taken among *CDC45, CDC6, CDT1, SLD5, MCM2* and *MCM7* are over-expressed (T/N>5, black lines) or not (T/N<5, gray lines) in the primary tumors. 25%S: time of 75% survival. i: incidence.

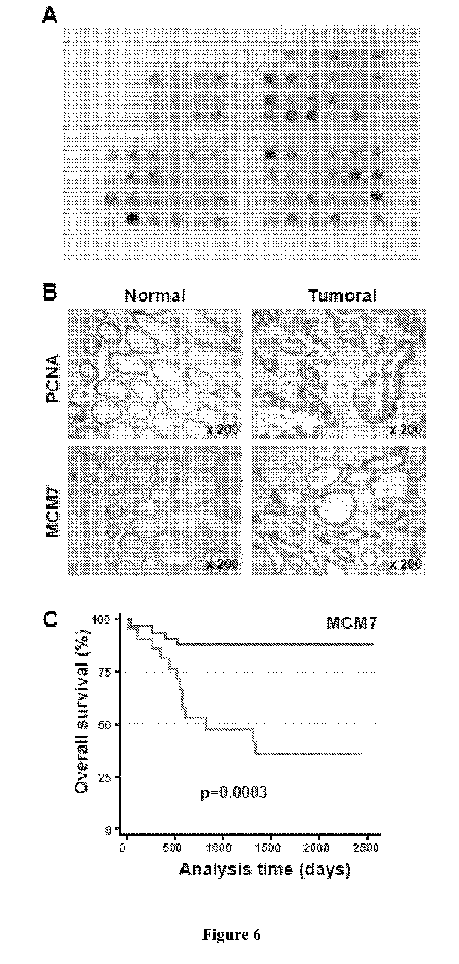

**Figure 6****. Incidence of the MCM7 expression level on overall survival.** Protein expression was analyzed using TMAs consisting of 74 pairs of cancer and adjacent normal mucosa samples. Two different cores (1mm in diameter) from both tumor (right) and normal (left) tissue were removed from paraffin-embedded tissue blocks that were prepared at the time of resection (A). They were then immunostained with anti-PCNA and anti-MCM7antibodies (B). (C) Kaplan Meier survival graphs related to 69 patients who were divided into 2 groups: the lowest tercile (group1, individuals whose percentage of immuno-stained cells was lower than 40%, light grey line) and the highest tercile (group2, individuals whose percentage of immuno-stained cells was higher than 72%, dark grey line). To compare these survival curves, we used the log-rank test. This test calculates a p-value testing the null hypothesis that the survival curves are identical in the two populations.

### EXAMPLES

### EXEMPLE 1. Determination of a "DNA replication" signature of progression and negative outcome in colorectal cancer

To clarify the importance of DNA replication in colorectal adenocarcinomas, we have analyzed the variation of expression of 47 DNA replication genes in tumor versus adjacent normal colorectal tissues and compared these data with disease progression and clinical features.

### 1.1. Materials and methods

### Patients and Methods

We selected 74 patients who underwent surgery for primary colorectal adenocarcinoma at the Toulouse Hospital between 1999 and 2002. The eligibility criteria for inclusion were availability of frozen normal and malignant tissues for each patient and possibility to obtain high-quality RNA and DNA. Exclusion criteria were treatment with adjuvant therapy and presence of tumor microsatellite instability (MSI). Histo-pathological and clinical findings were scored according to the tumor-node-metastasis (TNM) international staging system. MSI status was assessed by PCR amplification and anti-hMLH1, -hMSH2 and -hMSH6 immunostaining. Before RNA and DNA extraction, a frozen section was cut and stained with hematoxylin and eosin to evaluate the percentage of cancer cells. Malignant cells accounted for at least 50 % of the tumor area. Adjacent normal tissues were only mucosa. The observation time was defined as the interval between surgical resection and last contact. Data were censored at the last follow-up for living patients and for those who had died. The minimal duration of follow-up was 36 months and the mean duration 43 months. The study was approved by the National Institute of Cancer (INCa) following the recommendations of the National Agency of Agreement and Evaluation for Health (ANAES). Tumor samples were collected in agreement with the 2004 French Bioethics law.

### Quantification of gene expression

For RNA extraction, 40 to 80 5 to 10µm thick frozen sections from tumor and adjacent normal tissues were at once homogenized in the lysis buffer of the RNeasy extraction kit and total RNA was extracted according to the manufacturer's instruction (Qiagen, Courtaboeuf, France). The quality of total RNA was assessed with the Agilent 2100^{®} bio-analyzer using the RNA Nano Lab^{®} chip and RNA 6000 Nano Assay kit (Agilent Technologies, Santa Clara, CA). 1 to 2µg of total RNA were reverse transcribed using the High-Capacity cDNA Archive Kit (Applied Biosystems, Foster City, CA). All studied genes and four control genes (*18S, GAPDH, HPRT, YWHAZ*) were amplified in triplicate from tumor and normal samples using the TaqMan Universal PCR Master Mix and the TaqMan Low Density Array technology (Applied Biosystems). PCR amplifications were performed with the TaqMan Low Density Array technology and the 7900HT fast real time PCR system. To normalize gene expression in the matched tumor and normal samples, the QBase software (http://medgen.ugent.be/qbase/) was used to test the expression stability of the control genes. For each sample pair, the two most stable control genes were used by QBase to normalize the expression of all transcripts.

### Tissue Microarrays (TMA)

Using a TMA tool (Beecher Instruments, Alphelys, Plaisir, France), we removed for each patient two tumors and two normal tissue cores (1mm in diameter) from paraffin-embedded tissue blocks which were prepared at the time of resection. Immunostaining was performed on paraffin sections from TMA. Antibodies were anti-PCNA (Dako, Glostrup, Denmark) and anti-MCM7 (Abcam Inc., Cambridge, UK). After antigen retrieval and quenching of endogenous biotin, sections were incubated at room temperature for 60 minutes with the primary antibodies. Antibody binding was detected using the iVIEW ^{™} detection system (Ventana, Illkirch, France). Slides were analyzed using a workstation (Spot Browser, Alphelys) by two pathologists (JS and MD). Staining was exclusively nuclear with all antibodies. The percentage of stained cells was evaluated in both cancer and normal tissues and the ratios between tumor and normal tissues were calculated for each patient.

### Statistical Analysis

The major criteria of analysis were the individual ratio between standardized tumor and adjacent normal tissue gene expressions. In order to take into account the non Gaussian distribution of normalized malignant versus normal gene expression ratios, non parametric statistics analyses were used. Binomial exact tests evaluated the significance of gene over- and under-expression. Correlation between genes was assessed with a non parametric Spearman's correlation. Comparison of expression levels in samples with different tumor stages was performed using a non parametric Wilcoxon's test. Log-rank tests were used to compare survival curves (including all censored observations with a minimal follow-up period of 3 years) between groups defined according to gene expression's modifications. The significance level was set for an overall table-wide p-value of 0.05, uncorrected p-value was shown systematically and multiple testing was taken into account according to the Benjamini-Krieger's procedure. All computations were performed using Stata 9.0 SE.

### 1.2. Results

### Subsets of DNA replication genes are deregulated in colorectal cancers

Microarray studies have shown that DNA replication genes are often poorly expressed in human tissues (NCBI GEO Data Sets). Therefore, we used a real-time PCR-based approach to generate gene expression profiles of 74 coupled primary colorectal carcinomas at different stages of progression (see following Table 3).

**Table 3. Clinical/histological characteristics of the patients**

| **Characteristics** | **No. of patients (n=74)** | **%** |
|---|---|---|
| Sex | | |
| male | 46 | 62,16 % |
| female | 28 | 37.84 % |
| | | |
| Age (median) | 71,8 (IQR: 64.2-77.4) (range 38,8-89,4) | |
| Location | | |
| right side of colon | 17 | 22.97 % |
| left side of colon | 26 | 35.14 % |
| rectum | 30 | 40.54 % |
| multiple | 1 | 1.35 % |
| | | |
| Differentiation | | |
| well or moderate | 69 | 94.24 % |
| poor | 5 | 6.76% |
| | | |
| Tumor (T) stage | | |
| pT1 | 5 | 6.76% |
| pT2 | 18 | 24.32% |
| pT3 | 34 | 45.95% |
| pT4 | 17 | 22.97% |
| | | |
| Nodal (N) status | | |
| negative | 40 | 54.05% |
| positive | 34 | 45.95% |
| | | |
| Distant Metastasis (M) | | |
| none detected | 54 | 72.97 % |
| present | 20 | 27.03 % |

| Stage | | Overall Survival after 3 years |
|---|---|---|
| I | 18 (24.32 %) | 88.89 % [62.42 % ; 97.10 %] |
| II | 19 (25.68 %) | 89.47 % [64.08 % ; 97.26 %] |
| III | 17 (22.97%) | 64.71 % [37.71 % ; 82.34 %] |
| IV | 20 (27.03 %) | 42.22 % [20.45 % ; 62.60 %] |

| | | |
|---|---|---|
| Overall survival Log-Rank Test for homogeneity over Stages p=0.0031 | | |

We selected 47 genes known as playing a role in DNA synthesis, intra-S phase checkpoint signaling, or processing of DNA damage in the course of DNA elongation. We then identified which of these genes were up- or down-regulated in tumors (T) compared to adjacent control tissues (N). Deregulated genes were stratified in two groups based on the number of tumors in which each gene was either over- or under-expressed (see following Table 4). As expected both the "replicative" DNA polymerase/primase *POL A,* which is the only DNA polymerase involved in DNA replication and not in DNA repair synthesis, and the proliferative marker *PCNA* were over-expressed. Conversely, the DNA replication-independent *p57* gene, which is known as down-regulated in colon cancer, was mostly under-expressed (Table 4).

**Table 4. Differential gene expression in tumors and adjacent normal tissues**

| **3R gene** | **3R families** | **n** | **Over-expression** | **Under-expression** | **p** |
|---|---|---|---|---|---|
| PCNA | replication/repair | 73 | 52 | 21 | 0,0004 |
| POLA (POLα) | replication | 74 | 49 | 25 | 0,0070 |
| POLQ (POLθ) | replication /repair | 72 | 60 | 12 | 0,0000 |
| POLD1 (POLδ) | replication /repair | 73 | 40 | 33 | 0,4827 |
| POLE (POLε) | replication /repair | 74 | 35 | 39 | 0,7275 |
| POLB (POLβ) | replication /repair | 67 | 38 | 29 | 0,3284 |
| POLG (POLγ) | replication/repair | 74 | 16 | 58 | 0,0000 |
| POLH (POLη) | replication /repair | 74 | 21 | 53 | 0,0003 |
| POLI (POLτ) | replication /repair | 74 | 9 | 65 | 0,0000 |
| POLK (POLκ) | replication /repair | 74 | 27 | 47 | 0,0265 |
| POLL (POLλ) | replication /repair | 74 | 17 | 57 | 0,0000 |
| POLM (POLµ) | replication /repair | 63 | 27 | 36 | 0.3140 |
| REV3 (POLζ) | replication /repair | 74 | 8 | 66 | 0,0000 |
| REV 1 | replication /repair | 74 | 30 | 44 | 0,1301 |
| CDC45 | initiation | 74 | 57 | 17 | 0,0000 |
| CDC6 | initiation | 74 | 68 | 6 | 0,0000 |
| CDC7 | initiation | 74 | 52 | 22 | 0,0006 |
| CDT1 | initiation | 73 | 60 | 13 | 0,0000 |
| GEMININ | initiation | 74 | 18 | 56 | 0,0000 |
| DBF4 | initiation | 74 | 52 | 22 | 0,0006 |
| MCM2 | initiation | 74 | 63 | 11 | 0,0000 |
| MCM7 | initiation | 74 | 60 | 14 | 0,0000 |
| MCM8 | initiation/elongation | 74 | 50 | 24 | 0,0034 |
| SLD5 | initiation | 74 | 61 | 13 | 0,0000 |
| CYCE1 | initiation | 73 | 41 | 32 | 0,3491 |
| ORC2 | initiation | 74 | 42 | 32 | 0,2954 |
| ORC4 | initiation | 74 | 21 | 53 | 0,0003 |
| CHK1 | S-checkpoint | 74 | 61 | 13 | 0,0000 |
| CHK2 | S-checkpoint | 74 | 52 | 22 | 0,0006 |
| RAD17 | S-checkpoint | 74 | 12 | 62 | 0,0000 |
| RAD9 | S-checkpoint | 74 | 20 | 54 | 0,0000 |
| 53BP1 | S-checkpoint | 61 | 1 | 60 | 0,0000 |
| MCM9 | S-checkpoint | 62 | 14 | 48 | 0.0000 |
| ATM | S-checkpoint | 74 | 12 | 62 | 0,0000 |
| ATR | S-checkpoint | 74 | 40 | 34 | 0,5613 |
| ATRIP | S-checkpoint | 74 | 34 | 40 | 0,5613 |
| RAD51 | fork protection | 73 | 45 | 28 | 0,0604 |
| RUVBL1 | fork protection | 74 | 54 | 20 | 0,0001 |
| BLM | fork protection | 74 | 66 | 8 | 0,0000 |
| BRCA1 | fork protection | 74 | 54 | 20 | 0,0001 |
| BRCA2 | fork protection | 74 | 70 | 4 | 0,0000 |
| LIG1 | DNA break repair | 74 | 47 | 27 | 0,0265 |
| TIP60 | DNA break repair | 74 | 2 | 72 | 0,0000 |
| ERCC1 | fork resolution | 74 | 23 | 51 | 0,0015 |
| MUS81 | fork resolution | 74 | 4 | 70 | 0,0000 |
| ECO1 | cohesion | 74 | 25 | 49 | 0,0070 |
| P57 | CCR control | 74 | 17 | 57 | 0.0000 |

| | | | | | |
|---|---|---|---|---|---|
| The exact number of samples available for each analysis is shown as well as the number and the proportion of over- and under- expressing samples. P-values from bilateral exact binomial tests are given uncorrected; the significance level is evaluated using the Benjamini Krieger Yekutieli 2001 procedure for an overall FDR of 0.05. | | | | | |

Among the genes involved in "firing" of DNA replication (Fig. 1, Table 4), we observed that, most of them (i.e., *CDC45, CDC6, CDC7, CDTl, DBF4, MCM2, MCM7, MCM8* and *SLD5*) were over-expressed in tumors. In contrast, *GEMININ,* the S-phase-dependent inhibitor of *CDT1,* was significantly under-expressed. We next assessed whether these modifications in gene expression were linked to a specific stage of the disease progression. We determined that *DBF4, CDC7* and *CYCE1* were significantly over-expressed mostly in early stage tumors, whereas *GEMININ* inhibition was significant in later stages (see following Table 5).

**Table S2. Differential gene expression according to the stratified progression of the disease**

| **3R genes** | **DNA transactions** | **Stage 1** | | **Stage 2** | | **Stage 3** | | **Stage 4** | |
|---|---|---|---|---|---|---|---|---|---|
| | | **+/- expr.¹** | **p bilat** | **+/- expr.²** | **p bilat** | **+/- expr.³** | **p bilat** | **+/- expr.⁴** | **p bilat** |
| PCNA | replication/repair | 12/5 | 0.143 | 16/3 | 0.004 | 13/4 | 0.049 | 11/9 | 0.824 |
| POL A (POL α) | replication | 12/6 | 0.237 | 17/2 | 0,001 | 11/6 | 0.001 | 9/11 | 0,823 |
| POLQ (POL θ) | replication/repair | 16/0 | 0.000 | 16/3 | 0.004 | 14/3 | 0.001 | 14/6 | 0.115 |
| POL D1 (POL δ) | replication /repair | 12/6 | 0.237 | 11/8 | 0.647 | 7/9 | 0.647 | 10/10 | 1.000 |
| POL (POL ε) | replication /repair | 9/9 | 1.000 | 10/9 | 1.000 | 9/8 | 1.000 | 7/13 | 0.263 |
| POL B (POL β) | replication /repair | 11/6 | 0.332 | 11/7 | 0,480 | 9/6 | 0.480 | 7/10 | 0,629 |
| POL G (POL γ) | replication /repair | 4/14 | 0.030 | 5/14 | 0.063 | 3/14 | 0.063 | 4/16 | 0.011 |
| POL H (POL η) | replication /repair | 6/12 | 0.240 | 6/13 | 0.167 | 6/11 | 0.332 | 3/17 | 0.002 |
| POL I (POL τ) | replication /repair | 2/16 | 0.000 | 3/16 | 0.004 | 3/14 | 0.012 | 1/19 | 0.000 |
| POL K (POL κ) | replication /repair | 6/12 | 0.240 | 8/11 | 0.647 | 7/10 | 0.629 | 6/14 | 0.115 |
| POL (POL λ) | replication /repair | 6/12 | 0.240 | 4/15 | 0.019 | 5/12 | 0.143 | 2/18 | 0.000 |
| POL M (POL µ) | replication /repair | 6/7 | 1.000 | 7/10 | 0.629 | 8/6 | 0.791 | 6/13 | 0.670 |
| REV3 (POL ζ) | replication /repair | 1/17 | 0.000 | 3/16 | 0.004 | 3/14 | 0.012 | 1/19 | 0.000 |
| REV1 | replication /repair | 6/12 | 0.240 | 10/9 | 1.000 | 8/9 | 1.000 | 6/14 | 0.115 |
| CDC45 | initiation | 16/2 | 0.000 | 15/4 | 0.019 | 11/6 | 0.332 | 15/5 | 0.041 |
| CDC6 | initiation | 18/0 | 0.000 | 18/1 | 0.000 | 15/2 | 0.002 | 17/3 | 0.002 |
| CDC7 | initiation | 14/4 | 0.030 | 14/5 | 0.063 | 11/6 | 0.332 | 13/7 | 0.263 |
| CDT1 | initiation | 16/1 | 0.000 | 14/5 | 0.063 | 15/2 | 0.002 | 17/3 | 0.002 |
| GEMININ | initiation | 5/13 | 0.100 | 7/12 | 0.359 | 4/13 | 0.049 | 2/18 | 0.000 |
| DBF4 | initiation | 15/3 | 0.010 | 13/6 | 0.167 | 11/6 | 0.332 | 13/7 | 0.263 |
| MCM2 | initiation | 18/0 | 0.000 | 15/4 | 0.019 | 14/3 | 0.012 | 16/4 | 0.012 |
| MCM7 | initiation | 16/2 | 0.000 | 15/4 | 0.019 | 14/3 | 0.012 | 15/5 | 0.041 |
| MCM8 | initiatiort/elongatio | 14/4 | 0.030 | 14/5 | 0.063 | 12/5 | 0.143 | 10/10 | 1.000 |
| SLD5 | initiation | 17/1 | 0.000 | 18/1 | 0.001 | 14/3 | 0.012 | 12/8 | 0.503 |
| CYCE1 | initiation | 14/3 | 0.012 | 11/8 | 0.647 | 10/7 | 0,629 | 6/14 | 0.115 |
| ORC2 | initiation | 11/7 | 0.480 | 11/8 | 0.647 | 11/6 | 0.332 | 9/11 | 0.823 |
| ORC4 | initiation | 3/15 | 0.010 | 7/12 | 0.359 | 7/10 | 0.629 | 4/16 | 0.011 |
| CHK1 | S-checkpoint | 17/1 | 0.000 | 16/3 | 0.004 | 14/3 | 0.012 | 14/6 | 0.115 |
| CHK2 | S-checkpoint | 14/4 | 0.003 | 13/6 | 0.167 | 14/3 | 0.012 | 11/9 | 0.823 |
| RAD17 | S-checkpoint | 2/16 | 0.001 | 5/14 | 0.063 | 3/14 | 0.012 | 2/18 | 0.000 |
| RAD9 | S-checkpoint 5/13 | 5/13 | 0.096 | 5/14 | 0.063 | 6/11 | 0.332 | 4/16 | 0.011 |
| 53BP1 | S-checkpoint | 0/14 | 0.000 | 0/17 | 0.000 | 1/13 | 0.001 | 0/16 | 0.000 |
| MCM9 | S-checkpoint | 2/11 | 0.022 | 5/12 | 0.143 | 6/9 | 0.607 | 1/18 | 0.000 |
| ATM | S-checkpoint | 4/14 | 0.030 | 3/16 | 0.004 | 2/15 | 0.002 | 3/17 | 0.002 |
| ATR | S-checkpoint | 10/8 | 0.814 | 13/6 | 0.167 | 9/8 | 1.000 | 8/12 | 0.503 |
| ATRIP | S-checkpoint | 9/9 | 1.000 | 12/7 | 0.359 | 6/11 | 0.332 | 7/13 | 0.263 |
| RAD51 | fork protection | 11/6 | 0.332 | 12/7 | 0.359 | 13/4 | 0.049 | 9/11 | 0.824 |
| RUVBL1 | fork protection | 15/3 | 0.008 | 16/3 | 0.004 | 11/6 | 0.332 | 12/8 | 0.503 |
| BLM | fork protection | 17/1 | 0.000 | 17/2 | 0.001 | 15/2 | 0.002 | 17/3 | 0.003 |
| BRCA1 | fork protection | 15/3 | 0.008 | 17/2 | 0.001 | 11/6 | 0.332 | 11/9 | 0.824 |
| BRCA2 | fork protection | 17/1 | 0.000 | 18/1 | 0.000 | 17/0 | 0,000 | 18/2 | 0.000 |
| LIG1 | DNA break repair | 16/2 | 0.001 | 13/6 | 0.167 | 10/7 | 0.629 | 8/12 | 0.503 |
| TIP60 | DNA break repair | 0/18 | 0.000 | 0/19 | 0.000 | 1/16 | 0.000 | 1/19 | 0.000 |
| ERCC1 | fork resolution | 4/14 | 0.031 | 5/14 | 0.064 | 8/9 | 1.000 | 6/14 | 0.115 |
| MUS81 | fork resolution | 2/16 | 0.001 | 1/18 | 0.000 | 1/16 | 0,000 | 0/20 | 0.000 |
| ECO1 | cohesion | 6/12 | 0.238 | 6/13 | 0.167 | 7/10 | 0.629 | 6/14 | 0.115 |
| P57 | CCR control | 3/15 | 0.008 | 5/14 | 0.064 | 6/11 | 0.332 | 3/17 | 0.003 |

| | | | | | | | | | |
|---|---|---|---|---|---|---|---|---|---|
| +/-: number of tumors prone to over-/under-expression of a DNA replication gene. ¹ n= 18 except *MCM9, POL* µ (n=13) *53BP1* (n=14), *PCNA, CDT1, CYCLIN E1, POL* β, *RAD51,* (n=17), *POL* θ (n=16); ² n= 19 except *MCM9, POL* µ *, 53BP1* (n=17), *POL* β and *REV1* (n=18); ³ n=17 except *POL* µ *, 53BP1* (n=14), *MCM9, POL* β (n=15), *POL* δ (n= 16) ; ⁴n=20 except *53BP1* (n=16), *MCM9, POL* µ (n=19), *POL* β (n=17).P-values from bilateral exact binomial tests are given uncorrected; the significance level is evaluated using the Benjamini Krieger Yekutieli 2001 procedure for an overall FDR of 0.05 Red and green labels highlight significant modification of expression: over-expression in red and under-expression in green. | | | | | | | | | |

Regarding the DNA polymerase family, while the expression of the replicative *POL D* and *POL* E was not significantly affected in tumors, we found a defective expression of the genes encoding the mitochondrial POL γ and the "non conventional" translesional (TLS) DNA polymerases POL η, τ, κ, λ and Rev3/ζ, (Table 4 and Fig. 2), with one exception, *POL Q,* which was significantly over-expressed mainly at early stages (Table 5).
We also found a lower expression of genes, which sense DNA damage occurring in S-phase such as *ATM, 53BP1, RAD17* and *RAD9* (Table 4, Fig. 3A) and, specifically in stage 4 tumors, *RAD9* (Table 5). The new *MCM2-8* family member *MCM9,* which is likely to play a role in an S-phase checkpoint pathway, was also down-regulated. In contrast, the S-phase checkpoint genes products which are involved in the downstream protection of the stalled DNA replication forks (*RAD51, RUVBL1, BLM, BRCA1, BRCA2*), *BRCA1* being significantly up-regulated at early stages (Table 5), as well as the S-phase signaling mediators, *CHK1* and *CHK2,* were over-expressed (Table 4).

Finally, genes involved in repair of replication-induced DNA breaks (*TIP 60*), cohesion of chromatids (*EC01*), resolution of replication intermediates (*ERCC1, MUS81*) were all repressed (Fig. 3C). *TIP60* and *MUS81* were down-regulated in almost all tumors at all stages (Table 5).

To investigate whether the misregulation of replication genes was related or not to the proliferating status of the cancer tissues, we used a non parametric Spearman's correlation test to compare the expression of 34 representative replication genes with the part of proliferating cells in tissues. The proliferation status was assessed in tumors by a parallel staining on tissue micro arrays of PCNA protein, a marker of S cells. Interestingly Fig. 4 indicates that the misregulation in tumors of *SLD5, MCM9, DBF4, CDC6, ERCC1, POLA, POLK, RAD9, 53BP1, BRCA2* as well as the TLS DNA polymerase *POLQ* genes were not dependent on the proliferation status. This parallel estimation of the fraction of cycling cells also showed that *MCM7* overexpression was only moderately associated with proliferation.

As an additional control, we also micro-dissected in a subset of cases the proliferating bottom third of the colon crypts and used this normal and proliferating epithelium to calculate the T/Nµ expression ratios, where Nµ is the gene expression in the micro-dissected normal adjacent tissue. We then tested whether the T/N and the T/Nµ were in agreement (see following Table 6). We found that among genes for which expression was not associated with the PCNA staining, most of them i.e. *SLD5, MCM9, DBF4, CDC6, RAD9, BRCA2* and *POL Q* can probably be still classifiable as mis-regulated, since the over- or down-expression observed in tumors by comparing with the whole normal mucosa (T/N) was also found by normalizing with corresponding micro-dissected proliferating epithelium (T/Nµ).

**Table 6. Agreement between T/Nµ and T/N ratios**

| | **T/Nµ** | | | | | | **T/N** | | | | | | **Number of cases in agreement** |
|---|---|---|---|---|---|---|---|---|---|---|---|---|---|
| | **21** | **34** | **46** | **54** | **56** | **76** | **21** | **34** | **46** | **54** | **56** | **76** | |
| **PCNA** | - | + | - | - | - | - | + | + | + | - | + | - | 3 |
| **GEMININ** | - | - | - | - | - | - | + | - | + | - | + | - | 3 |
| **RUVBLI** | + | + | + | - | + | + | + | - | + | + | + | + | 4 |
| **CDC7** | + | + | - | - | - | - | + | + | - | + | + | + | 3 |
| **CHK2** | + | + | - | - | + | - | | + | + | + | + | - | 4 |
| **EC01** | + | - | + | + | - | - | - | - | - | + | - | - | 4 |
| **POLH (POL**η**)** | + | + | + | - | - | - | + | - | - | - | + | - | 3 |
| **MCM2** | - | + | - | - | - | - | + | + | + | + | + | + | 1 |
| **MCM8** | - | + | + | - | + | - | + | - | - | - | + | - | 3 |
| **CHK1** | + | + | - | - | + | + | + | + | - | + | + | + | 5 |
| **POLI (POL**τ**)** | - | - | + | - | - | - | - | - | - | - | - | - | 5 |
| **CDT1** | Pre-annlification not validated | | | | | | + | + | + | + | + | + | ND |
| **BRCA1** | + | + | - | - | + | + | + | - | + | + | + | + | 3 |
| **BLM** | + | + | - | - | + | - | + | + | + | + | + | + | 3 |
| **MCM7** | + | + | - | - | + | - | + | - | + | + | + | + | 2 |
| **ATM** | + | + | + | + | + | - | - | - | - | - | - | - | 1 |
| **ORC4** | + | - | + | - | - | - | - | - | - | - | + | - | 3 |
| **POLL (POL)λ** | Pre-amplification not validated | | | | | | - | - | - | - | + | - | ND |
| **CDC45** | | | | | | | + | + | + | + | + | + | ND |
| **MUS81** | | | | | | | - | - | - | - | + | - | ND |
| **TIP60** | + | - | + | - | - | - | - | - | - | - | - | - | 4 |
| **RAD17** | + | + | + | - | - | - | - | - | + | - | - | - | 4 |
| **POLQ (POLθ)** | + | + | + | - | + | - | + | + | + | + | + | + | 4 |
| **SLD5** | + | + | - | - | + | + | + | + | + | + | + | + | 4 |
| **MCM9** | - | - | + | - | - | - | - | - | + | - | - | - | 6 |
| **DBF4** | + | + | + | - | + | - | + | + | + | + | + | + | 4 |
| **CDC6** | + | + | - | + | + | + | + | + | + | + | + | + | 5 |
| **ERCC1** | - | + | + | - | - | - | - | - | - | - | + | - | 3 |
| **POLA (POL**α**)** | Pre-amplification not validated | | | | | | + | - | - | + | + | + | ND |
| **POLK (POL**κ**)** | + | + | + | - | - | - | - | - | + | - | + | - | 3 |
| **RAD9** | - | - | - | - | - | - | - | - | + | - | + | - | 4 |
| **53BP1** | - | + | + | - | - | - | ND | - | - | - | - | - | 3 |
| **BRCA2** | + | + | + | - | + | - | + | + | + | + | + | + | 4 |

| | | | | | | | | | | | | | |
|---|---|---|---|---|---|---|---|---|---|---|---|---|---|
| T (tumor)/ Nµ (micro-dissected normal adjacent mucosa) and T (tumor)/ N (whole normal adjacent mucosa) mRNA expression ratios between normalized values were calculated. (+) and (-) mean that T/Nµ or T/N were ≥ 1 and <1, respectively. A subset of cases i.e. coupled biopsies n° 21, 34, 46, 54, 56 and 76 (Cf. Figs. 1-3) were analysed. To estimate whether the gene expression depends on the proliferation status we calculated the number of cases for which the T/Nµ and T/N data were in agreement. The closer to six the number of cases is, the less the gene deregulation is influenced by the proliferation status. ND: not determined. METHODS: Micro-dissection of the bottom 1/3 of colon crypt epithelia was performed on frozen sections by using the Arcturus^{®} Pixel II system. An average of 400 crypts was micro-dissected from 10 normal mucosa. Total RNA was extracted by the RNeasy extraction kit (Qiagen). After reverse transcription, *pre-amplification* of the cDNA target cDNA was performed to provide sufficient amount of specific amplicons (Taqman^{®} Pre-Amp Master Mix, Applied Biosystems). Genes were amplified in duplicate from tumors and micro-dissected crypt samples using the TaqMan^{®} Universal PCR Master Mix, the TaqMan LDA technology and the 7900HT fast real time PCR system (Applied Biosystems). To evaluate the uniformity of the pre-amplification step, we compared the gene expression levels between pre-amplified and non-pre-amplified samples. Targets not uniformly pre-amplified were discarded from the study. | | | | | | | | | | | | | |

### Clusters of DNA replication genes are concomitantly deregulated

We then investigated whether deregulation of a given DNA replication gene in a tumor could be concomitant to that of other DNA replication genes by using a non parametric Spearman's test. Expression of the MCM loader *CDT1* was correlated with that of *MCM2* (rho=0.787) and *MCM7* (rho=0.778). In contrast, expression of the elongating *MCM8* helicase was independent of that of the two firing genes *MCM2* (rho=0.444) and *MCM7* (rho=0.581).

We then found that expression defects of genes encoding the TLS polymerases POL β, POL κ, POL τ, POL η, POL λ, REV3/POL ζ and the TLS polymerase-associated scaffold REV1 were significantly correlated. In other terms when one of these seven "repair" DNA polymerases was under-expressed in a given colorectal tumor, the probability to observe a concomitant down-regulation of the others was high (all rho > 0.700). In contrast, genes encoding *POL* θ or the replicative DNA polymerases *POL* δ, *POL* ε and *POL* α did not belong to this cluster. Interestingly the expression of *POLQ* was concomitant to that of *MCM2* or *MCM7* (rho= 0.739).

Finally we observed a concomitant deregulation of some DNA damage sensors. Indeed, expressions of *ATM, RAD9* and *RAD17* were significantly correlated (all rho>0.600). On the other hand, expression of the kinases *CHK1* and *CHK2* was independent of that of other S phase-checkpoint genes (rho<0.400).

### Deregulated expression of POL Q and MCM7 is associated with poor prognosis

The ultimate goal of our study was to identify DNA replication genes whose expression level in tumors could be informative about the patient's survival. A log-rank test for equality of survivor functions indicated that when an important (T/N >5) over-expression of at least three genes involved in licensing/firing of DNA replication (taken among *CDC45, CDC6, CDT1, SLD5, MCM2* and *MCM7*) was associated with a similar excess (T/N>5) of the translesional DNA polymerase *POL Q,* which was generally over-expressed in our cohort (Table 4, Fig. 2), higher morbidity and higher incidence could be expected (from 0.00027 in patients with under-expression of these genes to 0.00073 in patients with strong over-expression) (Fig. 5, p=0.050).

In an attempt to analyze the deregulation of DNA replication genes at the protein level, we constructed a tissue microarray (TMA) containing the 74 cancer samples and neighboring normal mucosal tissues (Fig. 6A), and tested the immunoreactivity of anti-PCNA and anti-MCM7 antibodies (Fig. 6B). We then evaluated the percentage of stained cells in both cancer and normal tissues (data not shown) and observed a correlation between protein expression and RNA level when PCNA (p=0.013) and MCM7 (p=0.032) were over-expressed (data not shown). The TMA data indicated (Fig. 6C) that MCM7 was significantly over-expressed in patients with negative outcome (p=0.0003). Indeed, two years after surgery, only about 40 % of patients with MCM7-overexpressing tumors (i.e. including more than 72% MCM7 immuno-stained cells) were alive in comparison to about 90 % of those with tumors characterized by lower levels of MCM7 (i.e. with less than 40% marked cells).

### 1.3. Discussion

Beside the standard TNM classification used by pathologists, there is a clear lack of tools to accurately predict the clinical outcome of a colorectal tumor. For example, it is totally unknown why twenty to forty percent of patients with a stage II colorectal cancer (i.e., early cancer without metastasis at diagnosis) will rapidly worsen and die. Our study focuses on the specific expression in colorectal cancer of "DNA replication" genes involved in initiation of DNA replication, DNA damage signaling, stabilization or resolution of stalled DNA replication intermediates.

Our main finding related to the significant relationship between the overexpression of the MCM7 protein and a negative outcome could help clinicians to define which colorectal cancers may have a bad prognosis. MCM7 protein is also over-expressed in glioblastomas, astrocytomas, prostate cancers, and induces cervix tumors in transgenic mice. It is the only MCM family member whose promoter contains a MYC-binding site and whose up-regulation favors tumorigenesis in mice. Interestingly MCM7-positive tumor cells are correlated with tumor metastases in colorectal cancer (Nishihara et al., 2008). It has been shown in *Xenopus* that excess MCM7 could activate dormant origins, favouring re-replication and genetic instability. Several studies have reported that MCM proteins are more reliable for labeling proliferative cells than Ki67, the routine histopathological marker of cell proliferation. Hence a non-invasive assay has been already developed to identify colorectal cancer by detection of MCM2 in colonocytes retrieved from the fecal surface.

Beside MCM7, we also show in colorectal tumors a concomitant elevated expression of other genes involved in firing of DNA replication origins such as *CDT1, CDC6, CDC45, CDC7, DBF4, MCM2, MCM8* or *SLD5.* In contrast the *CDT1* inhibitor, *GEMININ,* was inhibited. Excess *CDT1* in mice promotes cancer (Arentson et al., 2002), and *CDT1* (and *CDC6*) over-expression is correlated with a poor prognosis in non-small-cell lung carcinoma (Karakaidos et al., 2004). *GEMININ* is also considered as a prognostic factor in breast and brain tumors probably because of its importance in the regulation of replication origins. In *Xenopus,* MCM8 was identified as an initiating helicase also involved in DNA elongation. We show here that human *MCM8* is over-expressed in colorectal cancer together with the *PCNA* processive factor, and that this up-regulation is independent on that of *MCM2-7.* Taken together these results suggest that over-expression of licensing/firing genes contributes to colorectal tumorigenesis. This could be linked to an increase in the number of active origins of replication (and therefore re-replication and aneuploidy), as already shown in yeast.

Conversely, the TLS DNA polymerases were mainly concomitantly down-regulated in our cohort of tumors. Aside from their role in translesion, TLS polymerases are also "specialized" in key DNA transaction repairs, such as Nucleotide Excision Repair for POL κ, Base Excision Repair for POL β and POL λ, Homologous Recombination Repair for POL η and Non Homologous Recombination Repair for POL λ and POL µ. We have also shown that naturally occurring non-canonical DNA structures, which induce genomic instability *in vivo* and are postulated to be responsible for breakpoint hotspots in cancer, are physiological substrates of both Pol η and Pol κ. Deregulation of POL κ and λ has already been observed in colon cancer. In addition loss of REV3/POL ζ expression promotes gross genetic instability. The repression of TLS polymerases could favor a "de-specialization" of both repair and TLS processes mediated by these enzymes, inducing genetic instability. The observed inhibition of genes involved in S-phase checkpoint could favor these genetic changes by inhibiting the rate-limiting recognition of DNA damage.

Dissimilarly from all other TLS polymerases, our data confirm (Kawamura et al., 2004) that the newly identified *POL Q* is significantly over-expressed in colorectal tumours. POL θ is the only DNA polymerase with a helicase domain and it can efficiently bypass endogenous lesions such as APsites and thymidine glycols (Seki et al., 2004), therefore it could play a role in conventional DNA replication. Our study reveals that, when associated to over-expression of at least three firing genes (among *MCM2, MCM7, SLD5, CDT1, CDC6* and *CDC45*), up-regulation of *POL Q* is independent on the proliferation status of the tumors and is a negative prognostic marker. Interestingly we showed that contrary to other representative A-, X- or Y-TLS, and to a smaller extent replicative DNA polymerases, excess *POL Q* is concomitant with the up-regulation of these "firing" genes in tumors (see following Table 7).

**Table 7. Correlation between the expression of Pol Q and firing genes**

| | **Pol** θ **(A-family)** | **Pol** α **(B-family)** | **Pol** δ **(B-family)** | **Pol** ζ **(A-family)** | **Pol** β **(X-family)** | **Pol** κ **(Y-family)** |
|---|---|---|---|---|---|---|
| **MCM2** | 0.81 | 0.56 | 0.60 | 0.22 | 0.28 | 0.16 |
| **MCM7** | 0.74 | 0.60 | 0.67 | 0.27 | 0.37 | 0.25 |
| **SLD5** | 0.72 | 0.39 | 0.42 | 0.13 | 0.24 | 0.08 |
| **CDT1** | 0.73 | 0.40 | 0.58 | 0.00 | 0.14 | 0.00 |
| **CDC6** | 0.74 | 0.35 | 0.39 | 0.00 | 0.02 | 0.00 |
| **CDC45** | 0.71 | 0.24 | 0.39 | 0.00 | 0.00 | 0.00 |

| | | | | | | |
|---|---|---|---|---|---|---|
| The correlation was assessed by using a non parametric Spearman's correlation test (rho coefficients). | | | | | | |

Finally we found that S-phase checkpoint genes such as the DNA damage sensors *ATM, 53BP1, RAD17* or *RAD9,* are inhibited whereas S-phase checkpoint mediators such as *BLM, CHK1, CHK2, BRCA1 or BRCA2,* which are involved in the protection of stalled replication forks, are mainly over-expressed. It was recently suggested that the number of active origins may be increased by modification of BRCA1 status. The development of a tumor being probably facilitated by an "at all costs" DNA replication without blockage of the cell cycle, we suggest, that down-regulation of *in trans* (*ATM, 53BP1, RAD17, RAD9*) together with over-expression of *in cis* pathways (*BRCA1, BRCA2*) may be a new sensitive signature of aberrant proliferation. This hypothesis is reinforced by the observation that *MCM9,* a new MCM family member, which regulate the licensing activity is also down-regulated.

To summarize, our data show that, in matched colorectal tumors, most of "replication" genes are misregulated. We present evidence that misexpression of many of these genes does not likely just reflect the proliferating stage of the tumor cells and is not just a signature of dividing cells. Indeed we found that it was not associated with the level of PCNA and that compared to dividing control tissues it was not dependent on the proliferation status of normal mucosa. Replication genes involved in DNA synthesis are found as up-regulated, whereas genes important for the cellular response to DNA damage are mainly down-regulated. These findings reinforce the recent hypothesis about the existence of a relationship between replication stress and tumor progression. Indeed hyper-replication (more origins, more stabilized replication forks) could induce more DNA damage that might lead to genetic instability in the absence of functional S-phase checkpoint or DNA repair pathways, as observed in our cohort. Alongside the identification of individual markers, our work thus suggests that subsets of "DNA replication" genes could be considered as useful cancer "metamarkers".

### REFERENCES

Arentson E, Faloon P, Seo J, Moon E, Studts JM, Fremont DH et al. (2002). Oncogenic potential of the DNA replication licensing protein Cdt1. Oncogene, 21, 1150-1158.
Karakaidos P, S T, Vassiliou LV, Zacharatos P, Kastrinakis NG, Kougiou D et al. (2004). Overexpression of the replication licensing regulators hCdt1 and hCdc6 characterizes a subset of non-small-cell lung carcinomas: synergistic effect with mutant p53 on tumor growth and chromosomal instability--evidence of E2F-1 transcriptional control over hCdt1. Am J Pathol, 165, 1351-1365.
Kawamura K, Bahar R, Seimiya M, Chiyo M, Wada A, Okada S et al. (2004). DNA polymerase theta is preferentially expressed in lymphoid tissues and up-regulated in human cancers. Int. J. Cancer, 109, 9-16.
Nishihara K, Shomori K, Fujioka S, Tokuyasu N, Inaba A, Osaki M et al. (2008). Minichromosome maintenance protein 7 in colorectal cancer: implication of prognostic significance. Int. J Oncol, 33, 245-251.
Seki M, Masutani C, Yang LW, Schuffert A, Iwai S, Bahar I et al. (2004). Highefficiency bypass of DNA damage by human DNA polymerase Q. EMBO J., 23, 4484-4494.

## Claims

1. A method for diagnosing aggressiveness of a colorectal cancer in a patient from a colorectal cancer sample of said patient, comprising:
a) measuring in vitro the expression level of genes in said patient colorectal cancer sample and in a healthy colorectal tissue sample, wherein said genes are: POLQ, CDC45, CDC6, CDT1, SLD5, MCM2 and MCM7.
b) calculating for each gene an expression level ratio of the expression level in said patient colorectal cancer sample to the expression level in said healthy colorectal tissue sample,
c) comparing each gene expression level ratio to a corresponding threshold value, and
d) diagnosing colorectal cancer aggressiveness if the ratios of POLQ and at least 3 other genes are superior to their corresponding threshold values.

2. The method of claim 1, wherein said expression level is measured at the mRNA level.

3. The method of claim 2, wherein said expression level is measured using quantitative PCR or nucleic acid microarray technology.

4. The method of claim 1, wherein said expression level is measured at the proteic level.

5. The method of claim 4, wherein said expression level is measured using specific antibodies, in particular using tissue microarray technology.

6. The method of any one of claims 1-5, wherein each gene threshold value is 5.

7. A microarray comprising at most 500 probes, at least 7 of which specifically hybridize to POLQ, CDC45, CDC6, CDT1, SLD5, MCM2 and MCM7.

8. A kit for diagnosing aggressiveness of a colorectal cancer in a patient from a colorectal cancer sample of said patient, comprising the microarray of claim 5 or amplification primers specific for POLQ, CDC45, CDC6, CDT1, SLD5, MCM2 and MCM7.

9. The kit of claim 8, further comprising a healthy colorectal tissue mRNA or cDNA sample.

10. A method for choosing a suitable treatment of colorectal cancer in a patient, comprising:
a) diagnosing or not aggressiveness of said colorectal cancer in said patient using the method of claim 1, and
b) adding adjuvant radiotherapy or chemotherapy to surgical treatment if said colorectal cancer is diagnosed as aggressive in step a).
